# FASCICULE DE BREVET EUROPEEN

(11) **EP 0 357 467 B1**
(45) Date de publication et mention de la délivrance du brevet: **08.12.1993**
(21) Numéro de dépôt: 89401509.8
(22) Date de dépôt: 01.06.1989
(51) Int. Cl.: C07C 237/46, A61K 49/04

(54) **Nouveaux composés triiodobenzéniques non ioniques iodés et produits de contraste les contenant**
Nichtionische Trijodbenzin-Verbindungen und diese enthaltende Kontrastmittel
Non-ionic tri-iodobenzene compounds, and contrast agents containing same

(30) Priorité: 02.06.1988 FR 8807369; 23.01.1989 FR 8900762
(43) Date de publication de la demande: 07.03.1990
(73) Titulaire: GUERBET S.A., F-93420 Villepinte (FR)
(72) Inventeur: Schaefer, Michel, F-91380 Chilly-Mazarin (FR); Dugast-Zrihen, Maryse, F-75013 Paris (FR); Guillemot, Michel, F-75116 Paris (FR); Doucet, Didier, F-93190 Livry-Gargan (FR); Meyer, Dominique, F-75013 Paris (FR)
(74) Mandataire: Le Guen, Gérard

(56) Documents cités:
- EP-A- 0 015 867
- EP-A- 0 083 964
- EP-A- 0 345 163
- WO-A-88/09328
- DE-A- 3 429 949

## Description

La présente invention concerne des composés utilisables dans des produits de contraste pour la radiographie.

On utilise depuis longtemps comme produits de contraste des composés iodobenzéniques présentant sur le noyau benzénique plusieurs atomes d'iode, en général 3 atomes d'iode par noyau benzénique, et divers autres substituants. Ces autres substituants sont des groupes pharmacologiquement acceptables qui permettent l'administration des composés chez l'homme et les animaux. Ces substituants sont d'une manière générale choisis, d'une part, pour conférer aux composés une hydrosolubilité suffisante en vue de l'administration de ces composés en solution aqueuse et, d'autre part, pour conférer à ces composés une tolérance suffisante par l'organisme humain.

A cet effet, on a proposé des structures non-ioniques, c'est-à-dire des dérivés iodobenzéniques possédant des substituants non-ioniques.

Ainsi, dans le brevet FR-A-2 053 037, on a proposé des composés carbamoyl iodobenzéniques contenant au total au moins un groupe N-hydroxy alcoyle et au moins deux groupes hydroxy.

Un composé illustrant cette classe est le métrizamide qui s'avère être toutefois d'une stabilité limitée.

La présente invention vise à fournir de nouveaux composés non-ioniques qui soient bien tolérés par l'organisme humain, très stables en solution aqueuse, qui présentent une forte solubilité dans l'eau et qui en solution aqueuse présentent une faible viscosité.

A cet effet, la présente invention a pour objet des composés de formule : 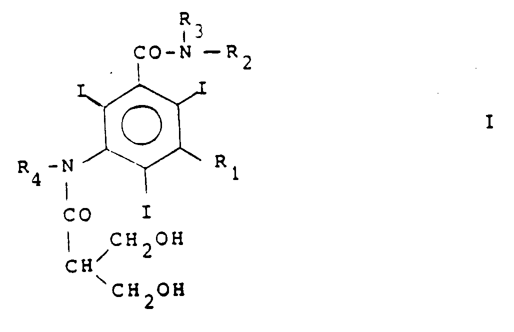
dans laquelle
R₁ représente un groupe de formule 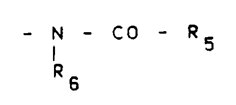
R₅ représentant un groupe alkyle en C₁-C₄, un groupe hydroxy alkyle en C₁-C₄ ou un groupe polyhydroxyalkyle en C₁-C₄,
R₆ représentant un atome d'hydrogène, un groupe alkyle en C₁-C₄, un groupe hydroxyalkyle en C₁-C₄ ou un groupe polyhydroxyalkyle en C₁-C₄,
ou un groupe de formule 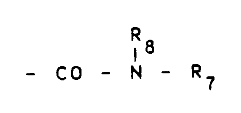
R₇ représentant un groupe hydroxyalkyle en C₁-C₄ ou un groupe polyhydroxyalkyle en C₁-C₄
R₈ représentant un atome d'hydrogène ou un groupe alkyle en C₁-C₄
R₂ représente un atome d'hydrogène, un groupe hydroxyalkyle en C₁₋C₄ ou un groupe polyhydroxyalkyle en C₁-C₄,
R₃ représente un atome d'hydrogène ou un groupe alkyle en C₁-C₄, et
R₄ représente un atome d'hydrogène, un groupe alkyle en C₁-C₄, un groupe hydroxyalkyle en C₁-C₄ ou un groupe polyhydroxyalkyle en C₁-C₄.

Dans la présente invention on désigne par groupe polyhydroxyalkyle un groupe polyhydroxyalkyle linéaire ou ramifié.

Un composé préféré de formule I est le composé de formule I dans laquelle
R₁ = -CO-NH-CH₂-CH₂OH, R₂ = -CH₂-CH₂OH, R₃ = H
R₄ = - CH₂-CHOH- CH₂OH.

Par ailleurs un groupe préféré de composés de formule I est celui constitué par les composés de type diamino-symétriques, c'est-à-dire les composés de formule 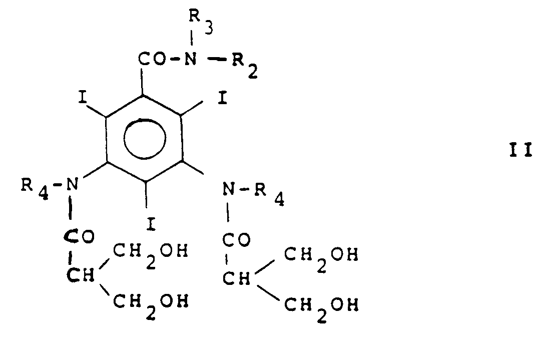
Les composés de formule I peuvent être préparés de manière classique notamment par des réactions d'acylation et/ou d'alkylation à partir de composés connus.

Ainsi les composés de type diaminosymétriques (composés de formule II) peuvent être préparés par
a) acylation d'un composé diamino de formule 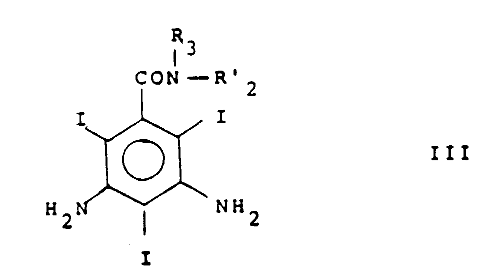 R₂′ représentant un groupe R₂ dont les groupes hydroxy ont été protégés, avec un chlorure d'acide de formule

   RCOCl IV

   dans laquelle R représente
   un groupe 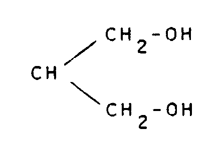 dont les groupes hydroxy sont protégés,
b) éventuellement alkylation du composé obtenu avec un réactif d'alkylation de formule

   R′₄Z V

   dans laquelle R′₄ a la signification donnée précédemment à l'exception de l'hydrogène et Z représente un groupe labile tel qu'un atome de chlore, de brome ou d'iode, en présence d'une base telle que méthylate de sodium, éthylate de sodium, hydrure de sodium ou hydroxyde de sodium,
c) déprotection.

Des composés de formule III sont décrits dans la demande française FR-A-2 614 299.

Les autres composés de formule III peuvent être préparés de manière analogue à partir notamment d'un 3,5-dinitrobenzoate d'alkyle de formule 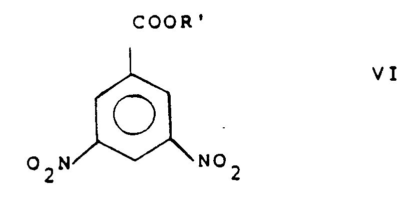
dans laquelle R′ est un groupe alkyle en C₁-C₄ tel qu'un groupe méthyle.

Les composé diamino dissymétriques peuvent être préparés également à partir d'un composé de formule VI par :
a) réaction avec une amine de formule 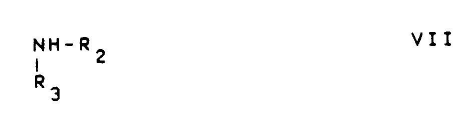 de façon à obtenir une composé de formule 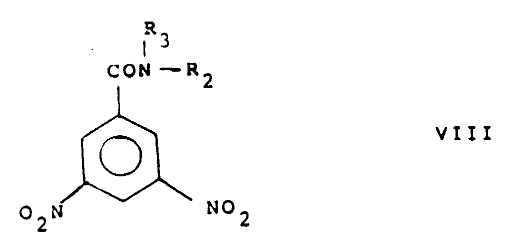
b) réduction par le sulfure d'ammonium de façon à obtenir un composé de formule 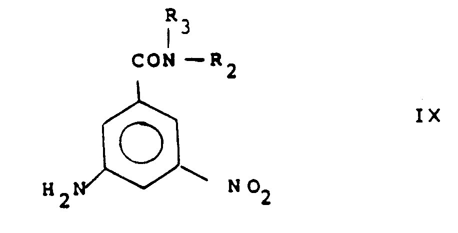
c) acylation du composé de formule IX par un chlorure d'acide de formule RCOCl (IV) de façon à obtenir une composé de formule 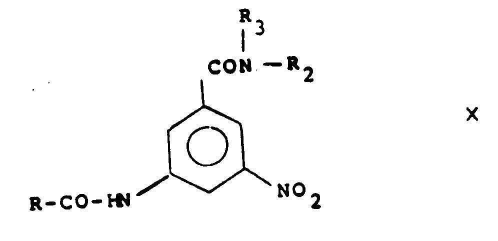
d) réduction et iodation du composé de formule X de façon à obtenir un composé de formule 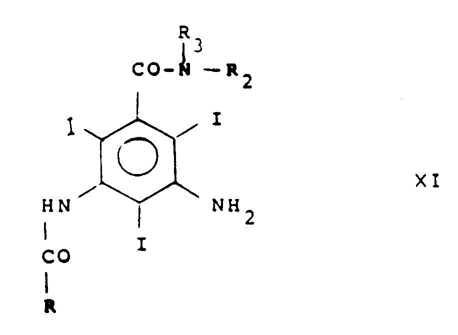
e) éventuellement alkylation d'un composé de formule XI avec un réactif d'alkylation de formule V de façon à obtenir un composé de formule 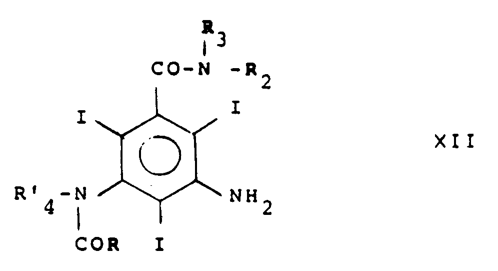
f) déprotection du composé de formule XII
g) acylation du composé déprotégé obtenu avec un chlorure d'acide de formule

   Cl-CO-R′₅ XIII

   R′₅ désignant un groupe R₅ dont les groupes hydroxy sont protégés de façon à obtenir après déprotection un composé de formule 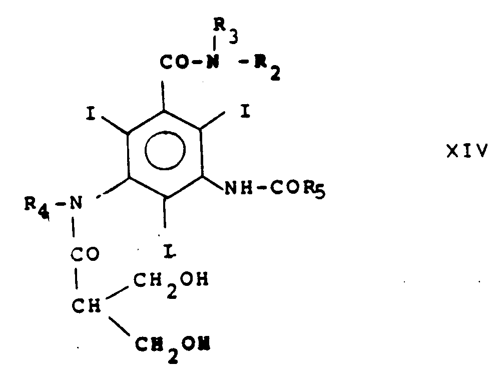 les étapes f et g pouvant être inversées, et éventuellement
h) alkylation pour obtenir un composé de formule I, dans laquelle R₆ est autre que l'hydrogène.

Les composés de type isophtaliques symétriques (composés de formule I dans laquelle 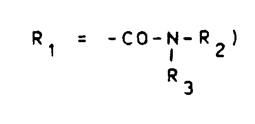
peuvent être préparés par
a) acylation d'une amine de formule 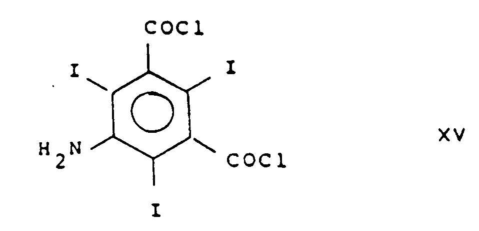 avec un chlorure d' acide de formule RCOCl (IV), de façon à obtenir un composé de formule 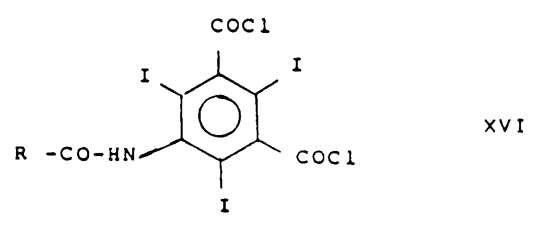
b) réaction sur le composé de formule XVI d une amine de formule 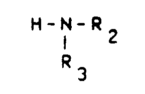 (VII) de façon à obtenir un composé de formule 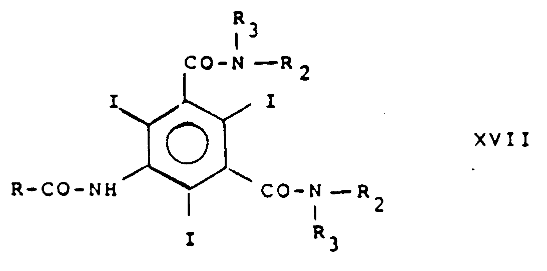 puis éventuellement soit
c) alkylation du composé de formule XVII avec un réactif d'alkylation de formule R′₄Z tel que défini précédemment et finalement
d) élimination des groupes protecteurs du groupe -CH(CH₂OH)₂, soit
e) élimination des groupes protecteurs du groupe -CH(CH₂OH)₂ et éventuellement
f) alkylation d'un composé déprotégé avec un réactif d'alkylation R′₄Z.

Les composés de type isophtaliques dissymétriques (composés de formule I dans laquelle 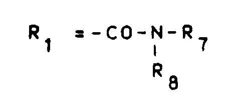
avec R₇≠R₂ et/ou R₈≠R₃) peuvent être préparé par
a) acylation d'une amine de formule 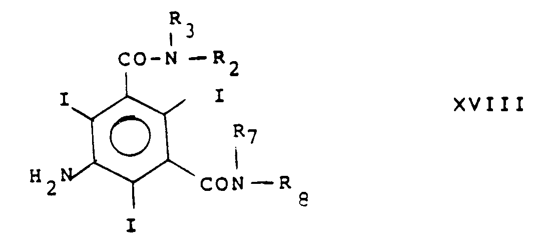 avec un chlorure d'acide de formule RCOCl,
b) éventuellement alkylation avec un réactif d'alkylation de formule R′₄Z et
c) élimination des groupes protecteurs du groups -CH-(CH₂OH)₂.

Les composés de formule XVIII peuvent être obtenus comme décrit dans EP-0 015 867.

En variante les composés de type isophtalique dissymétriques peuvent être préparés par
a) acylation d'une amine de formule 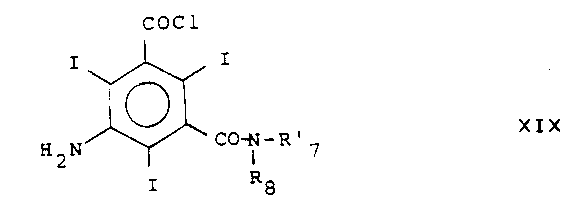 dans laquelle R′₇ représente un groupe R₇ dont les groupes hydroxy sont protégés,
   avec un chlorure d'acide de formule RCOCl (IV) de façon à obtenir un composé de formule 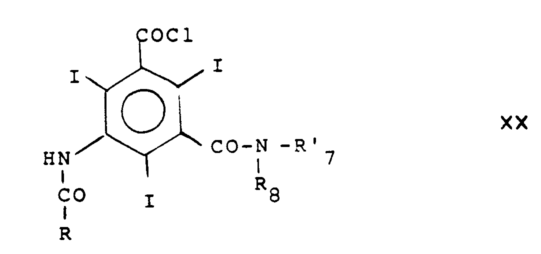
b) réaction sur le composé de formule XX d'une amine de formule 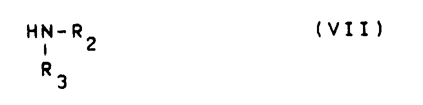 de façon à obtenir un composé de formule 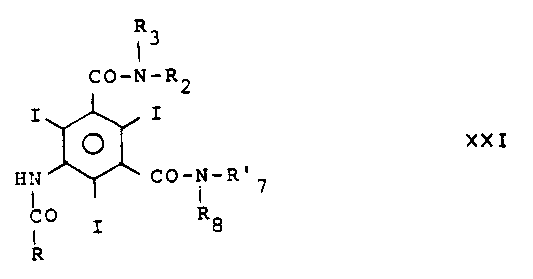 puis éventuellement soit
c) alkylation du composé de formule XXI avec un réactif d'alkylation de formule R′₄Z tel que défini précédemment et finalement
d) élimination des groupes protecteurs du groupe -CH(CH₂OH)₂ soit
e) élimination des groupes protecteurs du groupe -CH(CH₂OH)₂ et éventuellement
f) alkylation d'un composé déprotégé avec un réactif d'alkylation R′₄Z.

La présente invention a également pour objet des produits de contraste qui comprennent au moins un composé de formule I;

Ces produits de contraste sont utilisés chez l'homme et les animaux à des fins radiologiques.

La forme pharmaceutique préférée des produits de contraste selon l'invention est constituée par des solutions aqueuses des composés.

Les solutions aqueuses contiennent généralement un total de 5 à 100 g de composés de formule I pour 100 ml et la quantité injectable de telles solutions peut varier généralement de 1 à 1000 ml.

Les solutions aqueuses des composés de formule I peuvent également contenir certains additifs comme :
- du chlorure de sodium à des concentrations comprises entre 0,1 et 10 mM
- de l'EDTA disodique à des concentrations comprises entre 0,1 et 2 mM
- du citrate de sodium à des concentrations comprises entre 0,1 et 10 mM
- de l'héparine à des doses comprises entre 10 et 100 unités pour 100 ml de solution.

Ces compositions peuvent être administrées par toutes les voies classiquement utilisées pour les produits de contraste non ioniques iodés. Ainsi elles peuvent être administrées par voie entérale ou parentérale (voie intraveineuse, intraartérielle, opacification des cavités) et en particulier dans l'espace sous-arachnoidien.

On donnera ci-après un exemple de composition selon la présente invention.

| Composition | |
|---|---|
| Composé de l'exemple 1 | 65 g |
| Eau pour préparation injectable QSP | 100 ml |

Les exemples suivants illustrent la préparation des composés de formule I.

### EXEMPLE 1

### Préparation du 5-[3-hydroxy-2-(hydroxyméthyl)-N-(2,3-dihydroxypropyl)propionamido]-N′,N˝ -bis-(2-hydroxy-éthyl)-2,4,6-triiodo-isophtalamide.

### a) Préparation du dichlorure de 5-[2-isopropyl-1,3-dioxanne-5-carboxamido]-2,4,6-triiodo-isophtaloyle

137 g de chlorure de 5-amino-2,4,6-triiodo-isophtaloyle (0,23 mole) sont dissous dans 460 ml de DMAC auxquels sont ajoutés 110 g (0,57 mole) de chlorure d'acide 2-isopropyl-1,3-dioxanne-5-carboxylique. Le milieu réactionnel est maintenu 4 jours sous argon à température ambiante sous agitation. Le DMAC est éliminé sous vide. L'huile obtenue est extraite avec 3 l d'acétate d'éthyle et lavé 2 fois avec 1 l d'eau glacée. La phase organique est séchée et concentrée à sec. Le produit est cristallisé dans 200 ml de CH₂Cl₂. Après filtration, 110 g de solide sont obtenus :
Rdt : 64 %
CCM : SiO₂ CH₂Cl₂ Rf : 0,13
(60 F 254) SiO₂ éther/éther de pétrole 50/50
Rf : 0,52

### b) Préparation du 5-[2-isopropyl-1,3-dioxanne-5-carboxamido]-N′,N˝-bis-(2-hydroxy-éthyl)-2,4,6-triiodo-isophtalamide

130 g de produit obtenu en a (0,173 mole) sont dissous dans une solution de 750 ml de DMAC et 75 ml (0,534 mole) de triéthylamine. 33,7g d'éthanolamine (0,552 mole) sont additionnés goutte à goutte au milieu réactionnel. La réaction est ensuite laissée 3 heures à température ambiante sous agitation. Le chlorhydrate de triéthylamine est séparé par filtration et le DMAC est éliminé sous vide. L'huile obtenue est cristallisée dans 1 litre d'eau. Le produit est filtré et séché sous vide.
Rdt : 95 %
CCM : SiO₂ Rf : 0,25 CH₂Cl₂/méthanol 9/1
(60F254) SiO₂ Rf : 0,67 CH₂Cl₂/méthanol 8/2
% I : 45,6 (trouvé) - 47,6 (théorie)
Hypersil C8 5µ 15 cm
Pureté HPLC : 97 % NaH₂PO₄ 0,01M = 50
Méthanol = 50

### c) Préparation du 5-[3-hydroxy-2-(hydroxyméthyl)-N-(2,3-dihydroxypropyl)propionamido]-N′,N˝ -bis-(2-hydroxy-éthyl)-2,4,6-triiodoisophtalamide.

A une suspension de 100 g du produit obtenu en b (0,125 mole), dans 350 ml d'éthylène glycol sont ajoutés goutte à goutte 125 ml de méthylate de sodium 4N (0,5 mole) à 60°C puis 65 g de 1-chloro-2,3-propanediol (0,625 mole). Après 1 heure à 60°C, le milieu réactionnel prend en masse. 100 ml de méthylate de sodium 4N (0,4 mole) et 55,2 g de 1-chloro-2,3-propanediol (0,5 mole) sont ajoutés. L'ensemble est maintenu une nuit à 60°C. 31 ml de méthylate 4N (0,125 mole) et 20,7 g de 1-chloro-2,3-propanediol sont ajoutés à nouveau. L'agitation est maintenue 4 heures à 60°C. Les sels minéraux sont éliminés par filtration. L'éthylène glycol est évaporé sous vide.

Le résidu de distillation est repris avec 800 ml de HCl 10N et est laissé en agitation une nuit à température ambiante. Le milieu réactionnel est concentré à sec et résolubilisé dans 300 ml d'éthanol. Les sels minéraux sont éliminés par filtration. L'éthanol est évaporé sous vide et le résidu est cristallisé dans 1 litre d'alcool isopropylique. Le précipité est filtré et purifié par HPLC (RP 18)(élution eau).
Rdt total (alkylation-déprotection-purification): 52%
1) CCM (silice 60F254) : CH₂Cl₂/méthanol 7/3 Rf : 0,4
2)HPLC Hypersil C8 5µ 15 cm
   Tampon NaH₂PO₄ 0,01 M -- 97
   méthanol -- 3
   97 % en pureté
3) % I : 45,8 (trouvé) - 46,4 (théorie)
4) RMN (DMSO)
Massif, mal résolu, centré à 3,5 ppm (18 H); massif, centré à 4,5 ppm (OH) échangeable à D₂O (6 H); pic élargi à 8,4 ppm (NH) échangeable à D₂O (2 H).

### EXEMPLE 2 - Préparation du 5-glycolamido 3-[3-hydroxy-2-(hydroxyméthyl)-N-(2,3 dihydroxypropyl) propionamido]2,4,6-triiodo-N-hydroxyéthyl benzamide

### a) Préparation du 3,5-dinitro-N-(2-hydroxyéthyl)benzamide

750 g (3,32 moles) de 3,5 dinitro benzoate de méthyle sont mis en suspension dans 2 litres de méthanol en présence de 222,7g (3,65 moles) d'éthanolamine. Le mélange réactionnel est laissé 48 heures au reflux jusqu'à disparition de l'ester. Après 4 heures à température ambiante, le produit cristallisé est filtré, lavé avec 500 cm³ de chlorure de méthylène, puis séché à l'étuve à 60°C sous vide pendant 4 heures. Ce procédé permet de récupérer 718g de produit avec un rendement de 85%.
Point de fusion 140°C.
CCM (toluène/méthyléthyl cétone/acide formique (60/25/25) Rf : 0,5.

### b) Préparation du 3-nitro-5-amino-N-(2-hydroxyéthyl)benzamide

A une suspension de 25,5g (0,1 mole) de 3,5-dinitro-N-(2-hydroxyéthyl)benzamide dans 135 cm³ d'eau, sont additionnés à 70°C, 12,25g (0,18 mole) de sulfure d'ammonium. A la fin de l'addition le milieu d'abord homogène reprécipite après 1/2 heure à 70°C. On laisse le mélange réactionnel revenir à température ambiante et prolonge l'agitation durant 2 heures. Le précipité est filtré, lavé au méthanol (70 cm³) puis séché à l'étuve (60°C).
Masse obtenue 15,1g - Rendement 67%.
CCM (toluène/méthyléthyl cétone/acide formique 60/25/25). Rf : 0,3.
RMN du 1H (DMSO) : 3,4 ppm (massif; 4H,CH₂ aliphatiques);
4,65 ppm (massif, H échangeables au D₂O, NH₂): 5,9 ppm (singulet, H échangeable D₂O, OH); 7,4 - 7,7 ppm (2 multiplets; 3H, protons aromatiques), 8,6 ppm (massif, 1H, NH).

### c) Préparation du 3-nitro-5-[2-isopropyl 1,3-dioxanne-5-carboxamido] N-hydroxyéthyl benzamide

40g (0,177 mole) de 3-nitro-5-amino-N-(2-hydroxyéthyl)benzamide sont dissous dans 400 cm³ de DMAC. L'addition en présence de triéthylamine (54,6 cm³) de 74,9g (0,389 mole) du chlorure de l'acide 2-isopropyl-1,3-dioxanne-5-carboxylique produit un net effet thermique.

Le milieu réactionnel est maintenu 18 heures sous argon à température ambiante. Le milieu est filtré et le filtrat dilué avec de l'eau est extrait par de l'acétate d'éthyle. Le résidu obtenu après évaporation du solvant est traité par du carbonate de potassium (12g) dans 300 cm³ de méthanol. Après 48 heures d'agitation à température ambiante, le milieu est concentré puis est extrait par de l'acétate d'éthyle. Le produit brut obtenu après traitement est recristallisé dans un mélange éther/acétate d'éthyle 80/20. 37,8g de produit sont isolés avec un rendement de 56%. CCM (acétate d'éthyle Rf : 0,48).
HPLC Hypersil C8 5µ 15 cm.
Tampon pour NaH₂PO₄ 0,01 M 50%
MeOH 50%
Pureté : 94%.

### d) Préparation de 5-amino-3[2-isopropyl-1,3-dioxanne-5-carboxamido] 2,4,6-triiodo-N-hydroxyéthyl benzamide

Une solution méthanolique (1,4l) de 40g de 3-nitro-5-[2-isopropyl-1,3-dioxanne-5-carboxamido] N-hydroxyéthyl benzamide est agitée sous atmosphère d'hydrogène (5.10⁵ Pa) durant 5 heures à 50°C et en présence de 4g de charbon palladié. Le catalyseur est ensuite éliminé par filtration et le filtrat évaporé sous pression réduite. Le composé résultant est mis en suspension dans 950 cm³ d'eau. Le milieu s'homogénéise par addition de 20 cm³ d'acide chlorhydrique 2N. 63 cm³ de chlorure d'iode (à 70% en iode) sont ensuite introduits goutte à goutte sous forte agitation. Après 24 heures à température ambiante, le précipité est filtré, claircé à l'eau, repris dans l'éther. Après séchage on obtient 32g de produit soit un rendement de 42%.
CCM (dichlorométhane/méthanol 90/10) Rf : 0,8.

### e) Préparation de 5-amino-3[-N-(2,3 dihydroxypropyl)-2-isopropyl-1,3-dioxanne-5-carboxamido]-2, 4,6-triiodo-N-hydroxyéthyl benzamide

A une solution du composé obtenu en d) (20g 0,027 mole) dans un mélange éthylène glycol-diméthyl formamide v/v (160 ml) sont ajoutés goutte à goutte 84 cm³ de méthylate de sodium 4N (0,337 mole). Le milieu est porté à 60°C durant 1/2 heure, et à cette même température sont additionnés 36,1 cm³ de 1-chloro-2,3 -propanediol (0,432 mole). Le milieu réactionnel est maintenu 60 heures à 60°C sous azote. Les sels minéraux sont éliminés par filtration. L'éthylène glycol et le DMF sont évaporés sous vide. Le produit brut résultant est purifié sur silice silanisée (élution eau puis eau/méthanol 50/50). 16,5g de produit sont isolés. Rendement : 76%.
CCM (dichlorométhane/méthanol 80/20). Rf : 0,8.

### f) Préparation du 5-amino 3-[3-hydroxy-2(hydroxyméthyl)-N-(2,3-dihydroxypropyl)propionamido]-2,4,6-triido-N-hydroxyéthyl benzamide

16g (0,02 mole) du produit obtenu en e) sont déprotégés en présence de 80 cm³ d'acide chlorhydrique 10N, 48 heures à température ambiante. Après neutralisation et évaporation sous pression réduite, le résidu est précipité dans un mélange méthanol-éther (9/1), filtré puis purifié par HPLC (RP 18 (élution eau puis eau/méthanol 90/10).
4g de produit sont isolés avec un rendement total (déprotection, purification) de 30%.
CCM (dichlorométhane/méthanol 80/20). Rf : 0,25.
HPLC Hypersil C8 5µ 15 cm.
Tampon pour NaH₂PO₄ 0,01M 90%
MeOH 10%
Pureté : 97%.

### g) Préparation du 5-N-glycolamido-3-[3-hydroxy-2(hydroxyméthyl)-N-(2,3-dihydroxypropyl) propionamido]-2,4,6-triiodo-N-hydroxyéthyl benzamide

5,5g de chlorure de l'acide glycolique O-acétylé (0,04 mole) sont additionnés, goutte à goutte à température ambiante, à une solution de 3g du composé obtenu à l'étape f (0,004 mole) dans 30 cm³ de DMAC anhydre. Le milieu réactionnel est porté à 40°C pendant 12 heures, puis versé sur 250 cm³ d'eau glacée. Le précipité obtenu est filtré puis extrait à l'acétate d'éthyle. Après traitement puis évaporation, le produit obtenu dissous dans 50 cm³ de méthanol est déprotégé en présence de 10cm³ de soude 1N. La solution est maintenue sous agitation pendant 24 heures à température ambiante puis déminéralisée par passages successifs sur résines H⁺ (IRN77) et OH⁻ (IRN78). Après évaporation à sec le résidu est repris par l'éther éthylique, filtré puis séché.
masse obtenue 1,5g. Rendement global : 47%.
Pureté en iode : 99%.
CCM (acétate d'éthyle/méthanol/ammoniaque 60/40/1).
Rf : 0,25.
HPLC Hypersil C8 5µ 15 cm.
Tampon pour NaH₂PO₄ 0,01 M 90%
Me OH 10%
Pureté : 98%.

### EXAMPLE 3 - Préparation du 3,5-bis(3-hydroxy-2-(hydroxyméthyl propionamido)- 2,4,6-triiodo-N-(2,3-dihydroxypropyl) benzamide

### a) Préparation du 3-5-diamino-2,4,6-triiodo-N-(2,3-diacétoxypropyl) benzamide

301,5g (0,5 mole) de 3,5-diamino-2,4,6-triiodo-N-(2,3-dihydroxypropyl) benzamide sont mis en suspension dans 1l de pyridine anhydre refroidie vers 15°C. Après ajout de 2450 ml d'anhydride acétique, la solution est agitée 18h à température ambiante puis versée sur de l'eau acidulée. Après extraction à l'acétate d'éthyle, séchage de la phase organique et évaporation, on obtient 270g de produit avec un rendement de 78,5%.
Pureté en iode : 98,3%
CCM toluène/Méthyléthylcétone/HCOOH 60/25/35. Rf : 0,70.

### b) Préparation du 3,5-bis(2-isopropyl-1,3-dioxanne-5 carboxamido)-2,4,6-triiodo-N(2,3-diacétoxypropyl) benzamide

114,5 g (0,166 mole) du composé obtenu en a) sont dissous dans 350 ml de DMAC anhydre. L'addition de 128g (0,66 m) du chlorure de l'acide 2-isopropyl-1,3-dioxanne-5 carboxylique s'effectue à 0°C. Après une nuit d'agitation la masse réactionnelle est versée sur un mélange glace-eau. Le précipité est filtré, claircé à l'eau puis séché sous vide à 50°C.

### c) Préparation du 3,5-bis(2-isopropyl-1,3-dioxanne-5 carboxamido)-2,4,6-triiodo-N(2,3-dihydroxypropyl) benzamide

175 g du composé obtenu en b) en suspension dans 2,5l de méthanol sont agités à température ambiante en présence de 45g de carbonate de potassium, une nuit. Après évaporation de la masse réactionnelle le produit cristallise dans l'eau. Après filtration et séchage les cristaux obtenus avec 85% de rendement sont engagés directement dans l'étape suivante.

### d) Préparation du 3,5-bis(3-hydroxy-2-hydroxyméthyl propionamido)-2,4,6-triiodo-N-(2,3-dihydroxy propyl) benzamide

Le composé obtenu en c) est dissous dans 2l de HCl5N à 50°C. Après 18h d'agitation, la suspension obtenue est filtrée. Le filtrat est concentré sous vide et le résidu repris dans l'isopropanol.
108 g de produit cristallisé sont obtenus en 2 jets avec un rendement de 94%. CCM SiO₂ Butanol 60, eau 25, CH₃COOH 11 : RF : 0,2. Le produit est purifié par HPLC préparative sur SiO₂ RP18 15,25 µ éluant eau pure avec un rendement de 47%.
Pureté en iode 99,6%.
Pureté HPLC 99,1% (Hypersyl C₈ 5µ 15cm NaH₂PO₄ 0,01M 95, MeOH 5).
- RMN: ¹H 200 MHz (DMSO)
8,5 ppm (m,1H échangeable avec D₂O, O-CONH)
9,9 ppm (t,2H échangeable D₂O, O-NH-CO)
4,6 ppm (m,6H échangeable, OH)
3-4 ppm (m,13H, CH)
2,7 ppm (m,2H,NH-CH₂).

### EXEMPLE 4 - Préparation du 5-[3-hydroxy-2-(hydroxyméthyl)-N-(2-hydroxyéthyl)propionamido]-N-(2-hydroxyéthyl)-N#MIN#-(2,3-dihydroxypropyl)-2,4,6-triiodo-isophthalamide

### a) Préparation du chlorure de 5-(2-isopropyl-1,3-dioxanne-5-carboxamido)-2,4,6-triiodo-3-N′-(2-acétoxyéthyl) carbamoyl-benzoyle

5,36 g d'acide 2 isopropyl-1,3 dioxanne-5 carboxylique (0,0308 mole) sont dissous dans 18 ml de DMAC. Le milieu réactionnel est refroidi à 5°C et 2,55 ml de SOCl₂ (0,0350 mole) sont versés goutte à goutte de sorte que la température reste inférieure à 15°C. Après la fin de l'addition, le mélange réactionnel est laissé 3 heures à température ambiante.

Puis 6,0g de chlorure de 5-amino-2,4,6-triiodo-3-(N-2-acétoxyéthyl) carbamoyl-benzoyle (0,00906 mole) sont ajoutés. Le milieu réactionnel est maintenu sous argon 4 jours à température ambiante.

Le DMAC est éliminé sous vide. L'huile obtenue est reprise dans l'acétate d'éthyle; la phase organique est lavée à l'eau, séchée et concentrée à sec. Le produit est cristallisé dans 100ml d'éther.

Après filtration et séchage, 1,8g de produit sont obtenus, soit un rendement de 24%.
CCM (silice 60F 254) :
acétate d'éthyle/éther de pétrole 80/20 - Rf = 0,83.

### b) Préparation du 5-(2-isopropyl-1,3 dioxanne-5-carboxamido)-2,4,6-triiodo-N-(2-acétoxyéthyl)-N′-(2,3-dihydroxypropyl)isophthalamide

1g du produit obtenu en a (0,00122 mole) est dissous dans 100 ml de DMAC, puis 0,26 ml de triéthylamine (0,00189 mole) sont ajoutés. Au milieu réactionnel sont ajoutés goutte à goutte 0,18g de 3-amino-1,2-propanediol (0,00196 mole). Après l'addition, le mélange réactionnel est laissé sous agitation et sous argon à une température ambiante pendant 24 heures.

Le chlorhydrate de triéthylamine est filtré puis le DMAC évaporé. L'huile ainsi obtenue est cristallisée dans 20 ml d'éther.

Après filtration et séchage 0,8g de produit sont obtenus avec un rendement de 75,5%.
CCM (silice 60F254) : CHCl₃ /MeOH / NH₄OH 53 / 30 / 10 Rf = 0,77.

### c) Préparation du 5[3-hydroxy-2-(hydroxyméthyl)-N-(2-hydroxyéthyl)propionamido]-N-(2-hydroxyéthyl)-N′-(2,3-dihydroxy-propyl)-2,4,6-triiodoisophthalamide

0,4g du produit obtenu en b (0,000458 mole) est dissous dans 0,7 ml d'éthylène glycol et 0,69 ml d'une solution 4N de méthylate de sodium (0,00275 mole). A cette solution est ajouté 0,18 ml de chloroéthanol (0,00275 mole). Le milieu réactionnel est chauffé à 40°C pendant 5 heures. 0,34 ml de méthylate de sodium 4N et 0,1 ml de chloro-éthanol sont ajoutés.

L'ensemble est maintenu une nuit à 40°C.

Le pH du milieu réactionnel est ramené à 7,00 par addition d'acide chlorhydrique dilué.

L'éthylène glycol est évaporé sous vide.

Le résidu de distillation est repris avec 6ml d'eau et 5 ml d'acide chlorhydrique concentré puis est laissé en agitation une nuit à température ambiante.

Le milieu réactionnel est concentré puis purifié par HPLC préparative (RP 18, élution à l'eau). Après évaporation et séchage, on obtient 0,1g de produit, soit un rendement total (alkylation - purification) de 27%.
CCM (silice 60F254) : CH₂Cl₂/méthanol/7/3 - Rf = 0,33.
HPLC : colonne Hypersil C8 5µ 25 cm
NaH₂PO4 0,01M / MeOH : 95/S
pureté 95%
RMN¹H (Bruker - 200 MHz) dans DMSO : conforme à la structure attendue.

## Revendications (Revendications pour l'(les) Etat(s) contractant(s) suivant(s): AT, BE, CH, DE, FR, GB, GR, IT, LI, NL, SE)

1. Composés de formule 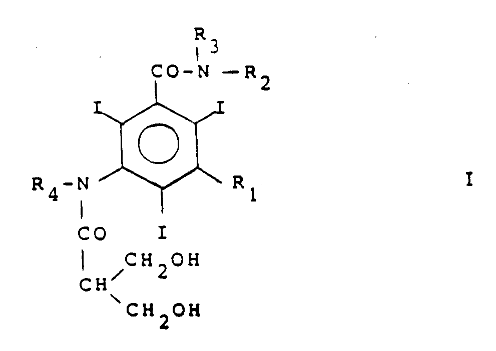 dans laquelle
R₁ représente un groupe de formule 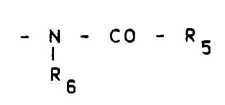 R₅ représentant un groupe alkyle en C₁-C₄, un groupe hydroxy alkyle en C₁-C₄ ou un groupe polyhydroxyalkyle en C₁-C₄,
R₆ représentant un atome d'hydrogène, un groupe alkyle en C₁-C₄, un groupe hydroxyalkyle en C₁-C₄ ou un groupe polyhydroxyalkyle en C₁-C₄,
ou un groupe de formule 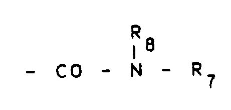 R₇ représentant un groupe hydroxyalkyle en C₁-C₄ ou un groupe polyhydroxyalkyle en C₁-C₄
R₈ représentant un atome d'hydrogène ou un groupe alkyle en C₁-C₄
R₂ représente un atome d'hydrogène, un groupe hydroxyalkyle en C₁₋C₄ ou un groupe polyhydroxyalkyle en C₁-C₄,
R₃ représente un atome d'hydrogène ou un groupe alkyle en C₁-C₄, et
R₄ représente un atome d'hydrogène, un groupe alkyle en C₁-C₄, un groupe hydroxyalkyle en C₁-C₄ ou un groupe polyhydroxyalkyle en C₁-C₄.

2. Composés selon la revendication 1 de formule 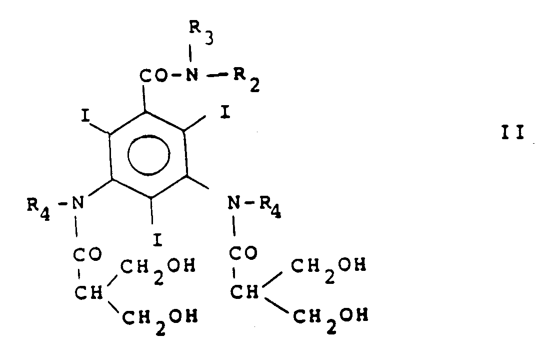 dans laquelle R₂, R₃ et R₄ ont les significations données à la revendication 1.

3. Composé selon la revendication 1 qui est le 5-[3-hydroxy-2-(hydroxyméthyl)-N-(2,3-dihydroxypropyl)propionamido]-N′,N˝-bis-(2-hydroxy-éthyl)-2,4,6-triiodoisophtalamide.

4. Composé selon la revendication 1 qui est le 5-glycolamido-3-[3-hydroxy-2-(hydroxyméthyl)-N-(2,3-dihydroxy-propyl)propionamido]-2,4,6-triiodo-N-hydroxyéthyl benzamide.

5. Composé selon la revendication 1 qui est le 3,5-bis(3-hydroxy-2-hydroxyméthyl propionamido-2,4,6-triiodo-N-(2,3-dihydroxypropyl) benzamide.

6. Composé selon la revendication 1 qui est le 5[3-hydroxy-2-(hydroxyméthyl)-N-(2-hydroxyéthyl)propionamido]-N-(2-hydroxyéthyl)-N′-(2,3-dihydroxypropyl)-2,4,6-triiodo-isophthalamide.

7. Produit de contraste caractérisé en ce qu'il contient au moins un composé selon l'une quelconque des revendications 1 à 6.

8. Produit de contraste selon la revendication 7, caractérisé en ce qu'il est constitué par une solution aqueuse du ou des composés.

## Revendications (Revendications pour l'(les) Etat(s) contractant(s) suivant(s): ES)

1. Procédé de préparation de composés de formule 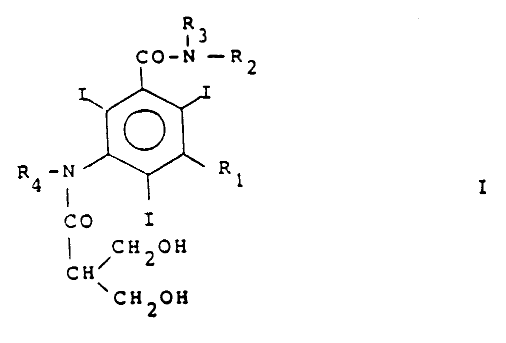 dans laquelle
R₁ représente un groupe de formule 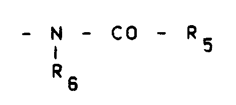 R₅ représentant un groupe alkyle en C₁-C₄, un groupe hydroxy alkyle en C₁-C₄ ou un groupe polyhydroxyalkyle en C₁-C₄,
R₆ représentant un atome d'hydrogène, un groupe alkyle en C₁-C₄, un groupe hydroxyalkyle en C₁-C₄ ou un groupe polyhydroxyalkyle en C₁-C₄,
ou un groupe de formule 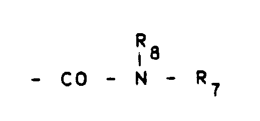 R₇ représentant un groupe hydroxyalkyle en C₁-C₄ ou un groupe polyhydroxyalkyle en C₁-C₄
R₈ représentant un atome d'hydrogène ou un groupe alkyle en C₁-C₄
R₂ représente un atome d'hydrogène, un groupe hydroxyalkyle en C₁₋C₄ ou un groupe polyhydroxyalkyle en C₁-C₄,
R₃ représente un atome d'hydrogène ou un groupe alkyle en C₁-C₄, et
R₄ représente un atome d'hydrogène, un groupe alkyle en C₁-C₄, un groupe hydroxyalkyle en C₁-C₄ ou un groupe polyhydroxyalkyle en C₁-C₄,
caractérisé en ce que l'on prépare ces composés par des réactions d'acylation et /ou d'alkylation.

2. Procédé selon la revendication 1 de préparation de composés de formule 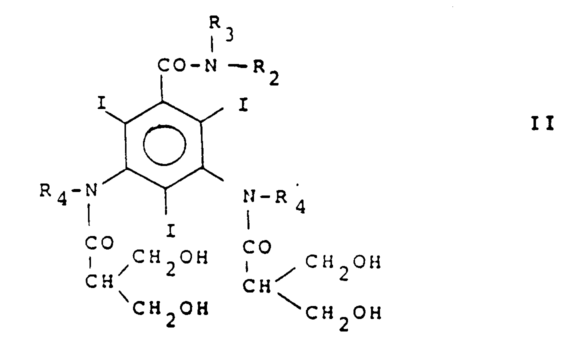 dans laquelle R₂, R₃ et R₄ ont les significations données à la revendication 1, caractérisé en ce que l'on effectue
a) une acylation d'un composé de formule 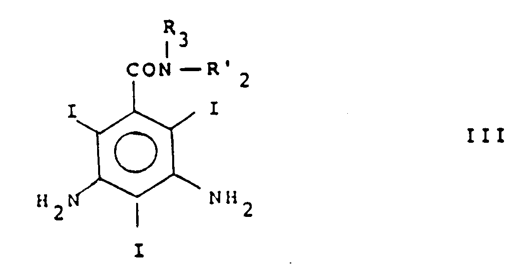 R₂′ représentant un groupe R₂ dont les groupes hydroxy ont été protégés, avec un chlorure d'acide de formule
RCOCl IV
dans laquelle R représente
un groupe 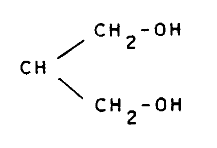 dont les groupes hydroxy sont protégés,
b) éventuellement une alkylation du composé obtenu avec un réactif d'alkylation de formule
R′₄Z V
dans laquelle R′₄ a la signification donnée précédemment à l'exception de l'hydrogène et Z représente un groupe labile tel qu'un atome de chlore, de brome ou d'iode, en présence d'une base telle que méthylate de sodium, éthylate de sodium, hydrure de sodium ou hydroxyde de sodium,
c) une déprotection.

3. Procédé selon la revendication 1 de préparation de composés de formule I dans laquelle R₁ est un groupe 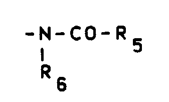 R₅ étant différent de -CH(CH₂OH)₂,
caractérisé en ce que l'on effectue
a) une réaction d'un composé de formule VI 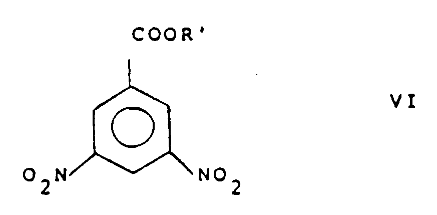 avec une amine de formule 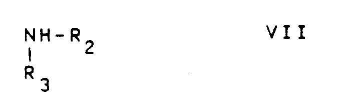 de façon à obtenir une composé de formule 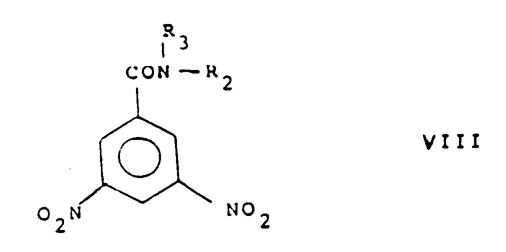
b) une réduction par le sulfure d'ammonium de façon à obtenir un composé de formule 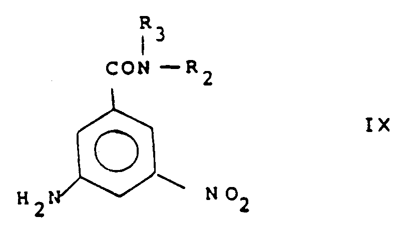
c) une acylation du composé de formule IX par un chlorure d'acide de formule RCOCl (IV) de façon à obtenir un composé de formule 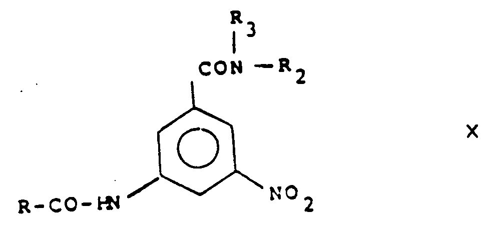
d) une réduction et iodation du composé de formule X de façon à obtenir un composé de formule 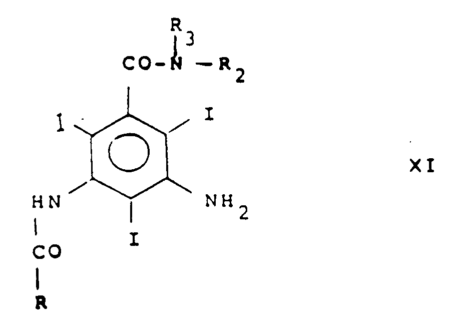
e) éventuellement une alkylation d'un composé de formule XI avec un réactif d'alkylation de formule V de façon à obtenir un composé de formule 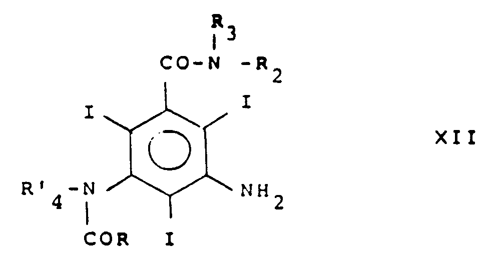
f) une déprotection du composé de formule XII
g) une acylation du composé déprotégé obtenu avec un chlorure d'acide de formule
Cl-CO-R′₅ XIII
de façon à obtenir un composé de formule 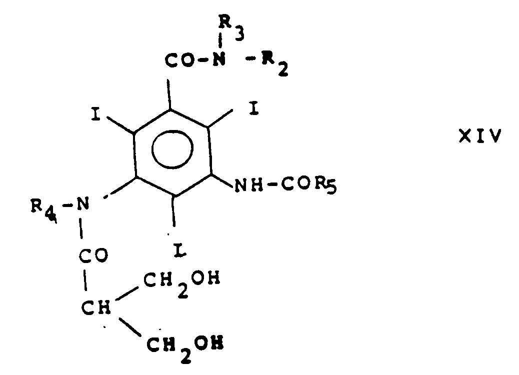 les étapes f et g pouvant être inversées, et éventuellement
h) une alkylation pour obtenir un composé de formule I, dans laquelle R₆ est autre que l'hydrogène.

4. Procédé selon la revendication 1 de préparation de composés de formule I dans laquelle 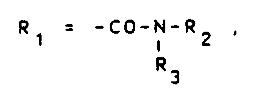 caractérisé en ce que l'on effectue
a) une acylation d'une amine de formule 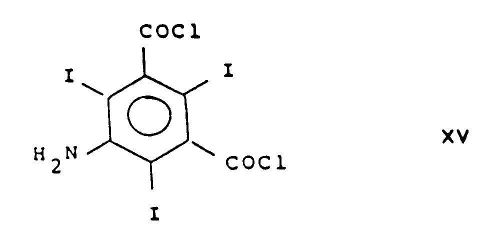 avec un chlorure d'acide de formule RCOCl (IV), de façon à obtenir un composé de formule 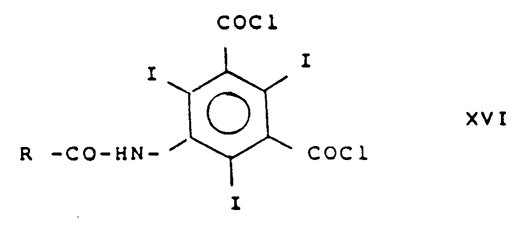
b) une réaction sur le composé de formule XVI d'une amine de formule 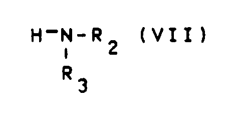 de façon à obtenir un composé de formule 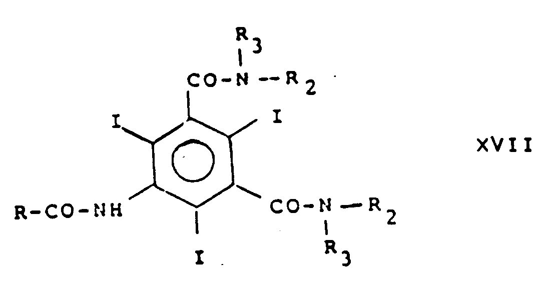 puis éventuellement soit
c) une alkylation du composé de formule XVII avec un réactif d'alkylation de formule R′₄Z tel que défini précédemment et finalement
d) une élimination des groupes protecteurs du groupe -CH(CH₂OH)₂ soit
e) une élimination des groupes protecteurs du groupe -CH(CH₂OH)₂ et éventuellement
f) une alkylation d'un composé déprotégé avec un réactif d'alkylation R′₄Z.

5. Procédé selon la revendication 1 de préparation de composés de formule I dans laquelle 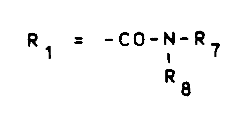 avec R₇ ≠ R₂ et/ou R₈ ≠ R₃ ,
caractérisé en ce que que l'on effectue
a) une acylation d'une amine de formule 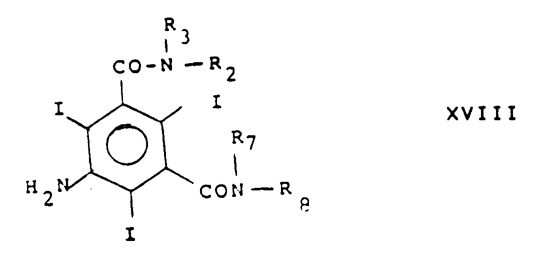 avec un chlorure d'acide de formule RCOCl,
b) éventuellement une alkylation avec un réactif d'alkylation de formule R′₄Z et
c) une élimination des groupes protecteurs du groupe -CH-(CH₂OH)₂.

6. Procédé selon la revendication 1 de préparation de composés de formule I dans laquelle 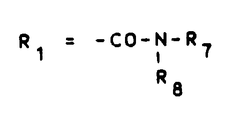 avec R₇ ≠ R₂ et/ou R₈ ≠ R₃ ,
caractérisé en ce que l'on effectue
a) une acylation d'une amine de formule 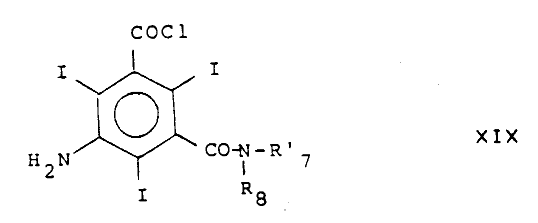 dans laquelle R′₇ représente un groupe R₇ dont les groupes hydroxy sont protégés,
avec un chlorure d'acide de formule RCOCl (IV) de façon à obtenir un composé de formule 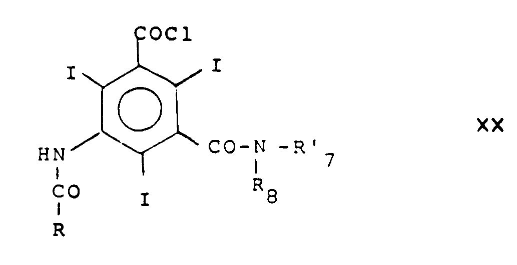
b) une réaction sur le composé de formule XX d'une amine de formule 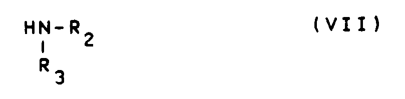 de façon à obtenir un composé de formule 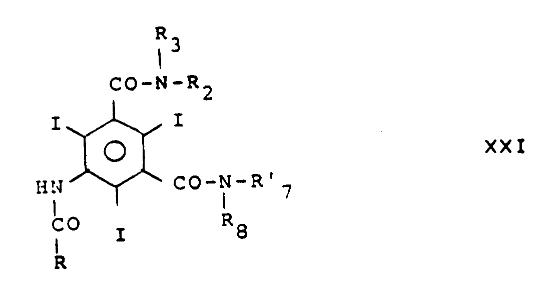 puis éventuellement soit
c) une alkylation du composé de formule XXI avec un réactif d'alkylation de formule R′₄Z tel que défini précédemment et finalement
d) une élimination des groupes protecteurs du groupe -CH(CH₂OH)₂ soit
e) une élimination des groupes protecteurs du groupe -CH(CH₂OH)₂ et éventuellement
f) une alkylation d'un composé déprotégé avec un réactif d'alkylation R′₄Z.

7. Procédé de préparation d'un produit de contraste, caractérisé en ce que l'on met un composé de formule I tel que défini à la revendication 1 sous une forme administrable.

## Claims (Claims for the following Contracting State(s): AT, BE, CH, DE, FR, GB, GR, IT, LI, NL, SE)

1. Compounds of formula 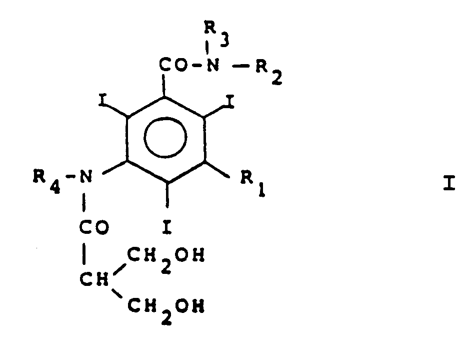 in which
R₁ represents a group of formula 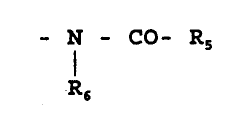 R₅ representing a C₁-C₄ alkyl group, a C₁-C₄ hydroxyalkyl group or a C₁-C₄ polyhydroxyalkyl group,
R₆ representing a hydrogen atom, a C₁-C₄ alkyl group, a C₁-C₄ hydroxyalkyl group or a C₁-C₄ polyhydroxyalkyl group, or a group of formula 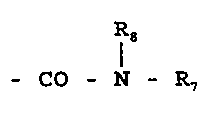 R₇ representing a C₁-C₄ hydroxyalkyl group or a C₁-C₄ polyhydroxyalkyl group
R₈ representing a hydrogen atom or a C₁-C₄ alkyl group
R₂ represents a hydrogen atom, a C₁-C₄ hydroxyalkyl group or a C₁-C₄ polyhydroxyalkyl group,
R₃ represents a hydrogen atom or a C₁-C₄ alkyl group, and
R₄ represents a hydrogen atom, a C₁-C₄ alkyl group, a C₁-C₄ hydroxyalkyl group or a C₁-C₄ polyhydroxyalkyl group.

2. Compounds according to Claim 1 of formula 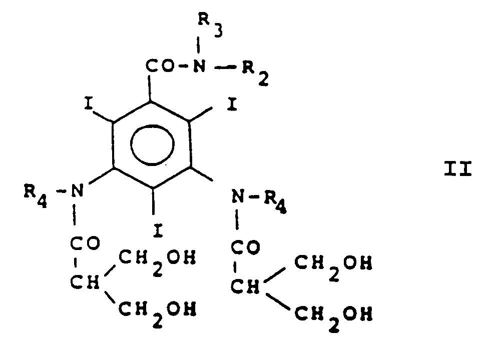 in which R₂, R₃ and R₄ have the meanings given in Claim 1.

3. Compound according to Claim 1 which is 5-[3-hydroxy-2-hydroxymethyl-N-(2,3-dihydroxypropyl)propionamido]-N',N''-bis(2-hydroxyethyl)-2,4,6-triidoisophthalamide.

4. Compound according to Claim 1 which is 5-glycolamido-3-[3-hydroxy-2-hydroxymethyl-N-(2,3-dihydroxypropyl)propionamido]-2,4,6-triiodo-N-(hydroxyethyl)benzamide.

5. Compound according to Claim 1 which is 3,5-bis(3-hydroxy-2-(hydroxymethyl)propionamido)-2,4,6-triiodo-N-(2,3-dihydroxypropyl)benzamide.

6. Compound according to Claim 1 which is 5-[3-hydroxy-2-hydroxymethyl-N-(2-hydroxyethyl)propionamido]-N-(2-hydroxyethyl)-N'-(2,3-dihydroxypropyl)-2,4,6-triidoisophthalamide.

7. Contrast agent, characterized in that it contains at least one compound according to any one of Claims 1 to 6.

8. Contrast agent according to Claim 7, characterized in that it consists of an aqueous solution of the compound(s).

## Claims (Claims for the following Contracting State(s): ES)

1. Process for the preparation of compounds of formula 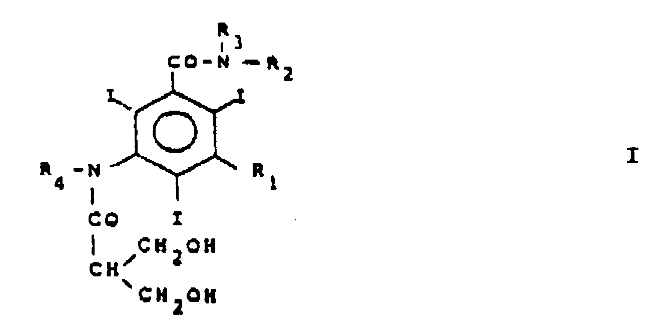 in which
R₁ represents a group of formula 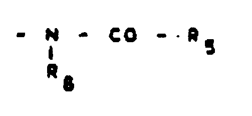
R₅ representing a C₁-C₄ alkyl group, a C₁-C₄ hydroxyalkyl group or a C₁-C₄ polyhydroxyalkyl group,
R₆ representing a hydrogen atom, a C₁-C₄ alkyl group, a C₁-C₄ hydroxyalkyl group or a C₁-C₄ polyhydroxyalkyl group,
or a group of formula 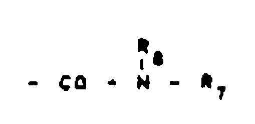
R₇ representing a C₁-C₄ hydroxyalkyl group or a C₁-C₄ polyhydroxyalkyl group
R₈ representing a hydrogen atom or a C₁-C₄ alkyl group
R₂ represents a hydrogen atom, a C₁-C₄ hydroxyalkyl group or a C₁-C₄ polyhydroxyalkyl group
R₃ represents a hydrogen atom or a C₁-C₄ alkyl group,
and
R₄ represents a hydrogen atom, a C₁-C₄ alkyl group, a C₁-C₄ hydroxyalkyl group or a C₁-C₄ polyhydroxyalkyl group,
characterized in that these compounds are prepared by acylation and/or alkylation reactions.

2. Process according to Claim 1 for the preparation of compounds of formula 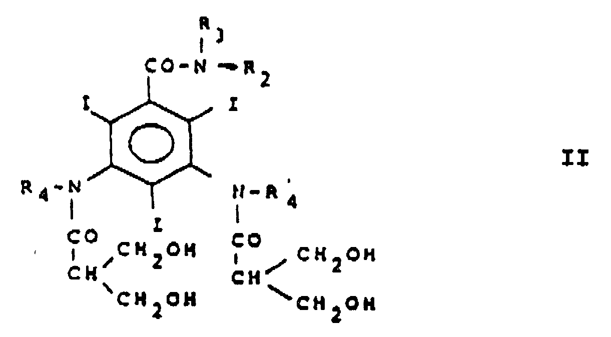 in which R₂, R₃ and R₄ have the meanings given in Claim 1, characterized in that
a) a compound of formula 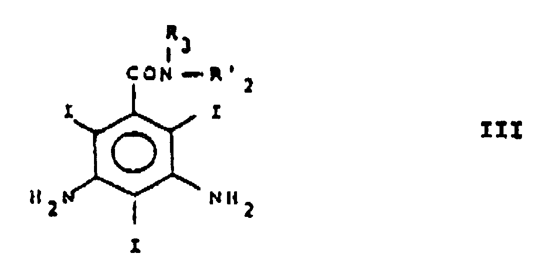 R'₂ representing a group R₂ in which the hydroxyl groups have been protected, is acylated with an acid chloride of formula
RCOCl IV
in which R represents a group 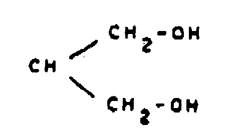 in which the hydroxyl groups are protected,
b) the compound obtained is optionally alkylated with an alkylating reagent of formula
R'₄Z V
in which R'₄ has the meaning given above except for hydrogen and Z represents a labile group such as a chlorine, bromine or iodine atom, in the presence of a base such as sodium methoxide, sodium ethoxide, sodium hydride or sodium hydroxide,
c) the compound obtained is deprotected.

3. Process according to Claim 1 for the preparation of compounds of formula I in which R₁ is a group 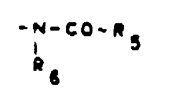
R₅ being other than -CH(CH₂OH)₂,
characterized in that
a) a compound of formula VI 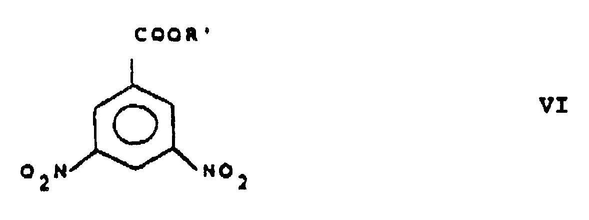 is reacted with an amine of formula 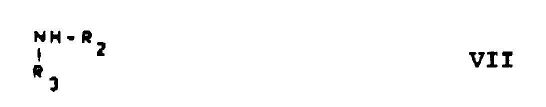 so as to obtain a compound of formula 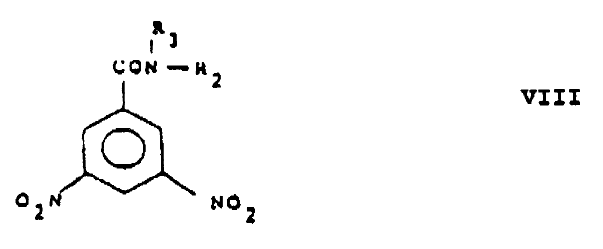
b) reduction is carried out with ammonium sulphide so as to obtain a compound of formula 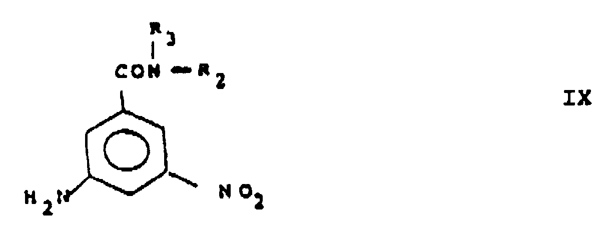
c) the compound of formula IX is acylated with an acid chloride of formula RCOCl (IV) so as to obtain a compound of formula 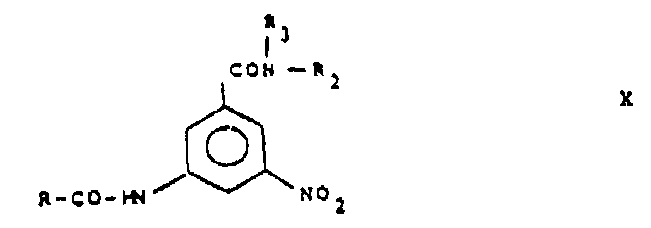
d) the compound of formula X is reduced and iodinated so as to obtain a compound of formula 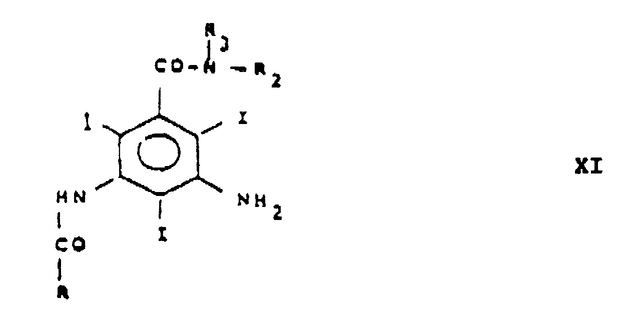
e) a compound of formula XI is optionally alkylated with an alkylating reagent of formula V so as to obtain a compound of formula 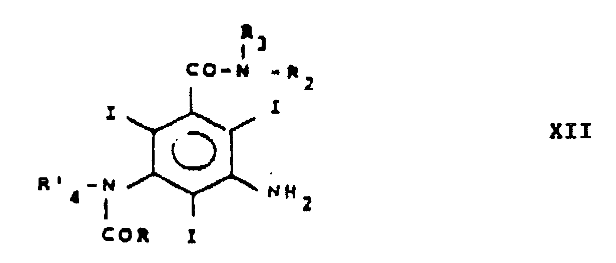
f) the compound of formula XII is deprotected
g) the deprotected compound obtained is acylated with an acid chloride of formula
Cl-CO-R'₅ XIII
so as to obtain a compound of formula 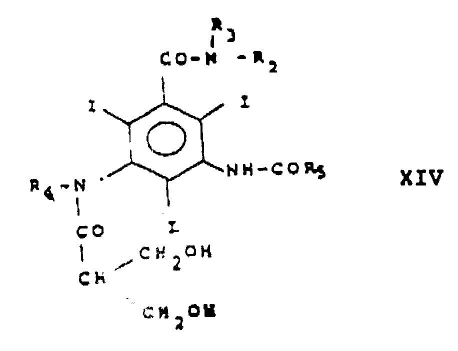 it being possible for Stages f and g to be reversed, and optionally
h) an alkylation is carried out to obtain a compound of formula I in which R₆ is other than hydrogen.

4. Process according to Claim 1 for the preparation of compounds of formula I in which 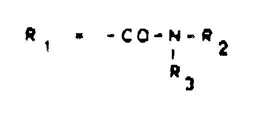 characterized in that
a) an amine of formula 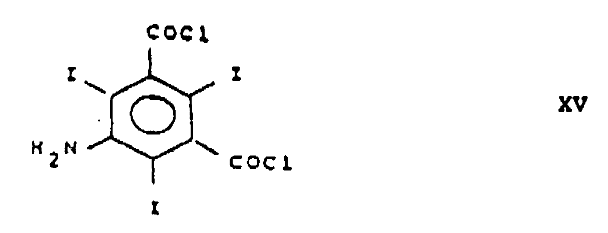 is acylated with an acid chloride of formula RCOCl (IV) so as to obtain a compound of formula 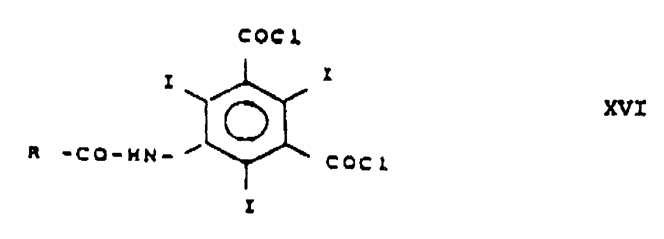
b) an amino of formula 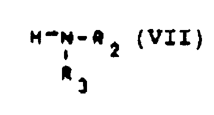 is reacted with the compound of formula XVI so as to obtain a compound of formula 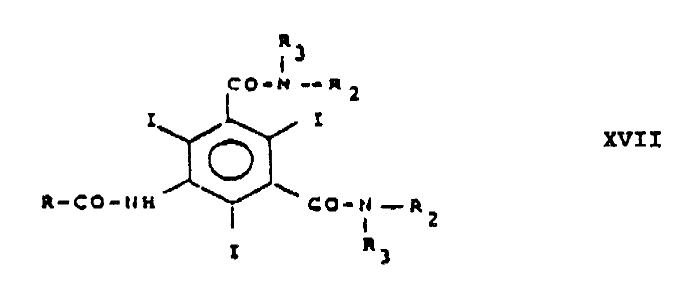 then optionally either
c) the compound of formula XVII is alkylated with an alkylating reagent of formula R'₄Z as defined above and finally
d) the protective groups of the -CH(CH₂OH)₂ group are removed or
e) the protective groups of the -CH(CH₂OH)₂ group are removed and optionally
f) a deprotected compound is alkylated with an alkylating reagent R'₄Z.

5. Process according to Claim 1 for the preparation of compounds of formula I in which 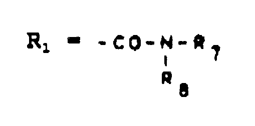 with R₇ ≠ R₂ and/or R₈ ≠ R₃,
characterized in that
a) an amine of formula 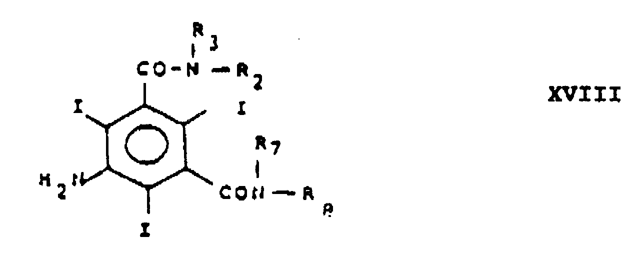 is acylated with an acid chloride of formula RCOCl,
b) an alkylation is optionally carried out with an alkylating reagent of formula R'₄Z and
c) the protective groups of the -CH(CH₂OH)₂ group are removed.

6. Process according to Claim 1 for the preparation of compounds of formula I in which 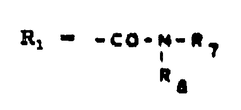 with R₇ ≠ R₂ and/or R₈ ≠ R₃,
characterized in that
a) an amine of formula 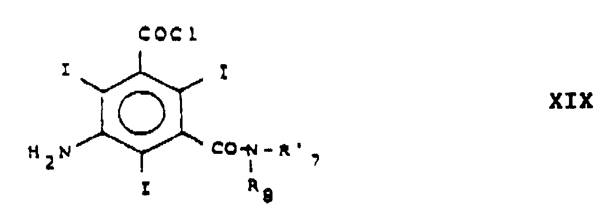 in which R'₇ represents an R₇ group in which the hydroxyl groups are protected,
is acylated with an acid chloride of formula RCOCl (IV) so as to obtain a compound of formula 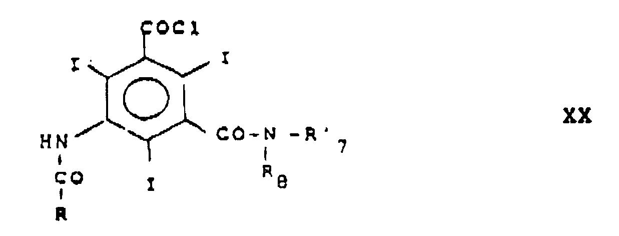
b) an amine of formula 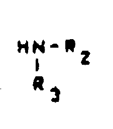 is reacted with the compound of formula XX so as to obtain a compound of formula 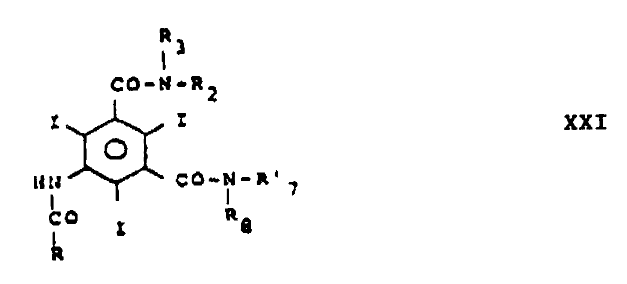 then optionally either
c) the compound of formula XXI is alkylated with an alkylating reagent of formula R'₄Z as defined above and finally
d) the protective groups of the -CH(CH₂OH)₂ group are removed or
e) the protective groups of the -CH(CH₂OH)₂ group are removed and optionally
f) a deprotected compound is alkylated with an alkylating reagent R'₄Z.

7. Process for the preparation of a contrast agent, characterized in that a compound of formula I as defined in Claim 1 is put into an administrable form.

## Patentansprüche (Patentansprüche für folgende(n) Vertragsstaat(en): AT, BE, CH, DE, FR, GB, GR, IT, LI, NL, SE)

1. Verbindungen der Formel 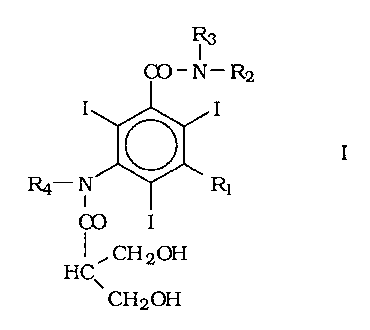 in der
R₁ eine Gruppe der Formel 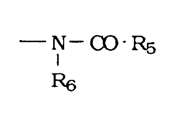 R₅ eine C₁-C₄-Alkylgruppe, eine C₁-C₄-Hydroxyalkylgruppe oder eine C₁-C₄-Polyhydroxyalkylgruppe,
R₆ ein Wasserstoffatom, eine C₁-C₄-Alkylgruppe, eine C₁-C₄-Hydroxyalkylgruppe oder eine C₁-C₄-Polyhydroxyalkylgruppe.
oder eine Gruppe der Formel 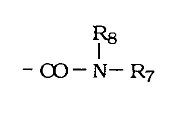 R₇ eine C₁-C₄-Hydroxyalkylgruppe oder eine C₁-C₄-Polyhydroxyalkylgruppe,
R₈ ein Wasserstoffatom oder eine C₁-C₄-Alkylgruppe,
R₂ ein Wasserstoffatom, eine C₁-C₄-Hydroxyalkylgruppe oder eine C₁-C₄-Polyhydroxyalkylgruppe,
R₃ ein Wasserstoffatom oder eine C₁-C₄-Alkylgruppe und
R₄ ein Wasserstoffatom, eine C₁-C₄-Alkylgruppe, eine C₁-C₄-Hydroxyalkylgruppe oder eine C₁-C₄-Polyhydroxyalkylgruppe bedeuten.

2. Verbindungen nach Anspruch 1 der Formel 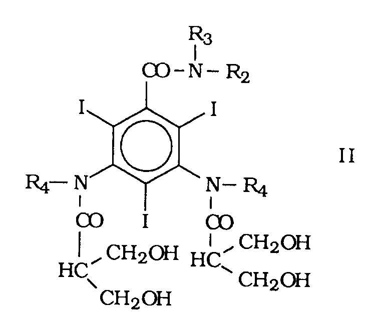 in der R₂, R₃ und R₄ die in Anspruch 1 angegebenen Bedeutungen besitzen.

3. Verbindung nach Anspruch 1, nämlich 5-[3-Hydroxy-2-(hydroxymethyl)-N-(2,3-dihydroxypropyl)-propionamido]-N',N''-bis(2-hydroxyethyl)-2,4,6-triiod-isophthalamid.

4. Verbindung nach Anspruch 1, nämlich 5-Glykolamido-3-[3-hydroxy-2-(hydroxymethyl)-N-(2,3-dihydroxypropyl)-propionamido]-2,4,6-triiod-N-hydroxyethyl-benzamid.

5. Verbindung nach Anspruch 1, nämlich 3,5-Bis(3-hydroxy-2-hydroxymethyl-propionamido-2,4,6-triiod-N-(2,3-dihydroxypropyl)-benzamid.

6. Verbindung nach Anspruch 1, nämlich 5-[3-Hydroxy-2-(hydroxymethyl)-N-(2-hydroxyethyl)-propionamido]-N-(2-hydroxyethyl)-N'-(2,3-dihydroxypropyl)-2,4,6-triiod-isophthalamid.

7. Kontrastmittel, **dadurch gekennzeichnet**, daß es mindestens eine Verbindung nach einem der Ansprüche 1 bis 6 enthält.

8. Kontrastmittel nach Anspruch 7, **dadurch gekennzeichnet**, daß es aus einer wäßrigen Lösung der Verbindung(en) besteht.

## Patentansprüche (Patentansprüche für folgende(n) Vertragsstaat(en): ES)

1. Verfahren zur Herstellung von Verbindungen der Formel 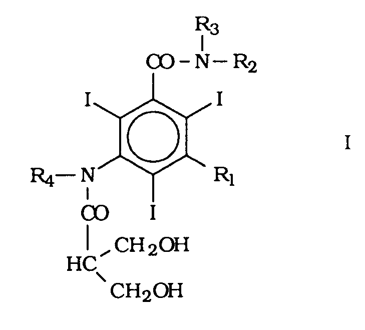 in der
R₁ eine Gruppe der Formel 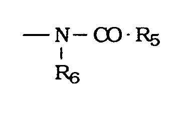 R₅ eine C₁-C₄-Alkylgruppe, eine C₁-C₄-Hydroxyalkylgruppe oder eine C₁-C₄-Polyhydroxyalkylgruppe,
R₆ ein Wasserstoffatom, eine C₁-C₄-Alkylgruppe, eine C₁-C₄-Hydroxyalkylgruppe oder eine C₁-C₄-Polyhydroxyalkylgruppe,
oder eine Gruppe der Formel 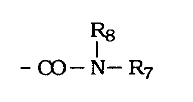 R₇ eine C₁-C₄-Hydroxyalkylgruppe oder eine C₁-C₄-Polyhydroxyalkylgruppe,
R₈ ein Wasserstoffatom oder eine C₁-C₄-Alkylgruppe,
R₂ ein Wasserstoffatom, eine C₁-C₄-Hydroxyalkylgruppe oder eine C₁-C₄-Polyhydroxyalkylgruppe,
R₃ ein Wasserstoffatom oder eine C₁-C₄-Alkylgruppe und
R₄ ein Wasserstoffatom, eine C₁-C₄-Alkylgruppe, eine C₁-C₄-Hydroxyalkylgruppe oder eine C₁-C₄-Polyhydroxyalkylgruppe bedeuten,
**dadurch gekennzeichnet**, daß man diese Verbindungen durch Acylierungs-und/oder Alkylierungs-Reaktionen herstellt.

2. Verfahren nach Anspruch 1 zur Herstellung von Verbindungen der Formel 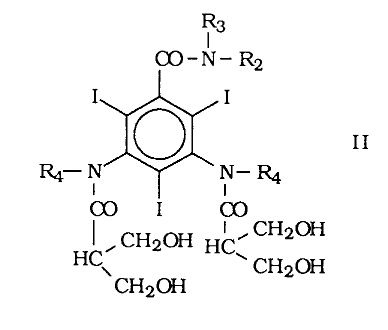 in der R₂, R₃ und R₄ die in Anspruch 1 angegebenen Bedeutungen besitzen, **dadurch gekennzeichnet**, daß man
a) eine Acylierung einer Verbindung der Formel 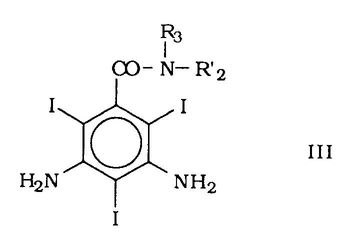 in der R'₂ eine Gruppe R₂ darstellt, deren Hydroxylgruppen geschützt sind, mit einem Säurechlorid der Formel
RCOCl IV
in der R eine Gruppe 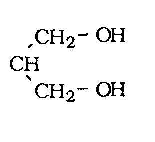 deren Hydroxylgruppen geschützt sind, bedeutet, bewirkt,
b) gegebenenfalls eine Alkylierung der erhaltenen Verbindung mit einem Alkylierungsreagens der Formel
R'₄Z V
in der R'₄ die oben angegebenen Bedeutungen besitzt, mit Ausnahme des Wasserstoffatoms und Z eine labile Gruppe, wie ein Chloratom, ein Bromatom oder ein Iodatom bedeutet, in Gegenwart einer Base, wie Natriummethylat, Natriumethylat, Natriumhydrid oder Natriumhydroxid, durchführt und
c) eine Abspaltung der Schutzgruppen bewirkt.

3. Verfahren nach Anspruch 1 zur Herstellung von Verbindungen der Formel I, in der R₁ eine Gruppe der Formel 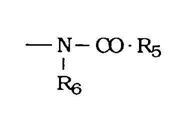 worin R₅ von -CH(CH₂OH)₂ verschieden ist,
**dadurch gekennzeichnet**, daß man
a) eine Umsetzung einer Verbindung der Formel VI 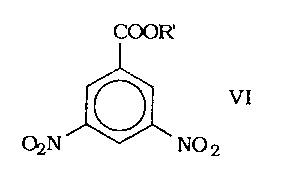 mit einem Amin der Formel 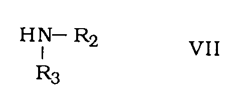 bewirkt zur Bildung einer Verbindung der Formel 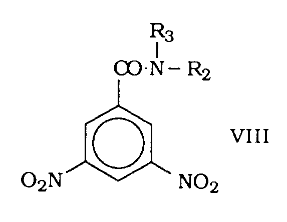
b) eine Reduktion mit Ammoniumsulfid bewirkt zur Bildung einer Verbindung der Formel 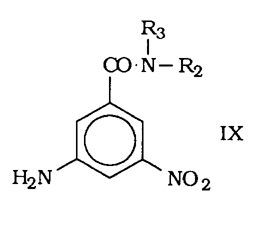
c) eine Acylierung der Verbindung der Formel IX mit einem Säurechlorid der Formel RCOCl (IV) durchführt zur Bildung einer Verbindung der Formel 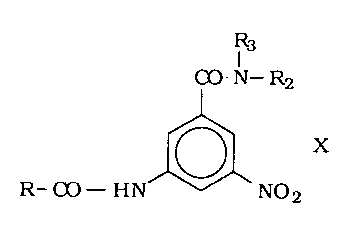
d) eine Reduktion und Iodierung der Verbindung der Formel X durchführt zur Bildung einer Verbindung der Formel 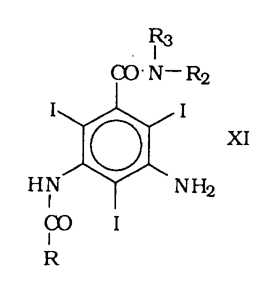
e) gegebenenfalls eine Alkylierung einer Verbindung der Formel XI mit einem Alkylierungsreagens der Formel V durchführt zur Bildung einer Verbindung der Formel 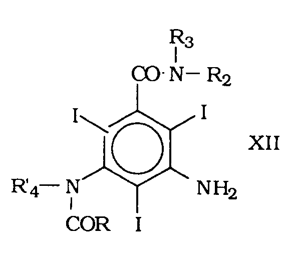
f) die Schutzgruppen der Verbindung der Formel XII abspaltet,
g) eine Acylierung der von den Schutzgruppen befreiten erhaltenen Verbindung mit einem Säurechlorid der Formel
Cl - CO - R'₅ XIII
durchführt zur Bildung einer Verbindung der Formel 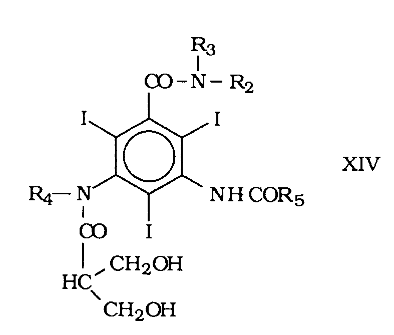 wobei die Stufen f) und g) auch in umgekehrter Reihenfolge durchgeführt werden können,
und gegebenenfalls
h) eine Alkylierung durchführt zur Bildung einer Verbindung der Formel I, worin R₆ von Wasserstoff verschieden ist.

4. Verfahren nach Anspruch 1 zur Herstellung von Verbindungen der Formel I, worin 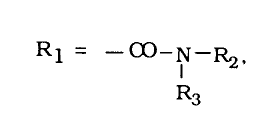 **dadurch gekennzeichnet**, daß man
a) eine Acylierung eines Amins der Formel 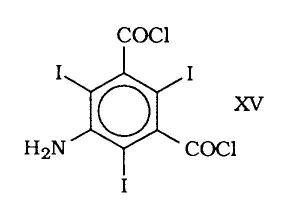 mit einem Säurechlorid der Formel RCOCl (IV) durchführt zur Bildung einer Verbindung der Formel 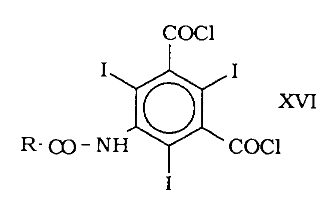
b) die Verbindung der Formel XVI mit einem Amin der Formel 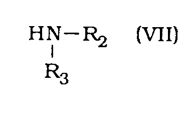 durchführt zur Bildung einer Verbindung der Formel 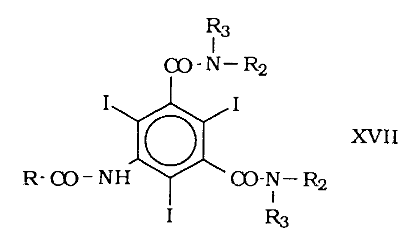 und dann gegebenenfalls entweder
c) eine Alkylierung der Verbindung der Formel XVII mit eine Alkylierungsreagens der Formel R'₄Z, wie sie oben definiert ist, durchführt und schließlich
d) die Schutzgruppen der Gruppe -CH(CH₂OH)₂ abspaltet, oder
e) eine Abspaltung der Schutzgruppen der Gruppe -CH(CH₂OH)₂ durchführt und gegebenenfalls
f) eine Alkylierung der von den Schutzgruppen befreiten Verbindung mit einem Alkylierungsreagens R'₄Z bewirkt.

5. Verfahren nach Anspruch 1 zur Herstellung von Verbindungen der Formel I, worin 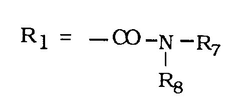 und R₇ ≠ R₂ und/oder R₈ ≠ R₃ sind,
**dadurch gekennzeichnet**, daß man
a) eine Acylierung eines Amins der Formel 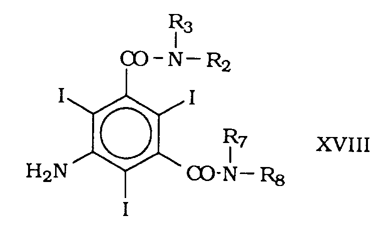 mit einem Säurechlorid der Formel RCOCl bewirkt,
b) gegebenenfalls eine Alkylierung mit einem Alkylierungsreagens der Formel R'₄Z durchführt und
c) die Schutzgruppen der Gruppe -CH-(CH₂OH)₂ abspaltet.

6. Verfahren nach Anspruch 1 zur Herstellung von Verbindungen der Formel I, in der 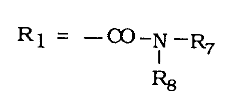 und R₇ ≠ R₂ und/oder R₈ ≠ R₃ sind,
**dadurch gekennzeichnet**, daß man
a) eine Acylierung eines Amins der Formel 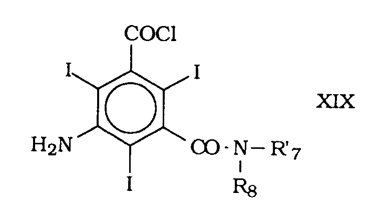 in der R'₇ eine Gruppe R₇ bedeutet, deren Hydroxylgruppen geschützt sind, mit einem Säurechlorid der Formel RCOCl (IV) bewirkt, so daß man eine Verbindung der Formel 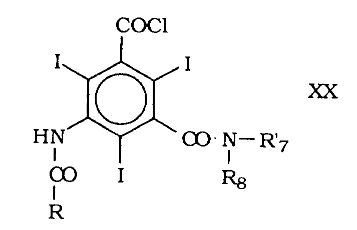 erhält,
b) die Verbindung der Formel XX mit einem Amin der Formel 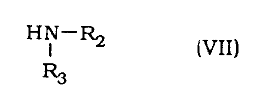 umsetzt, so daß man eine Verbindung der Formel 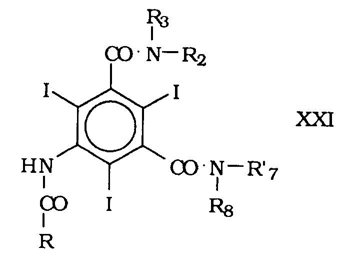 erhält, und gegebenenfalls entweder
c) eine Alkylierung der Verbindung der Formel XXI mit einem Alkylierungsreagens der Formel R'₄Z, wie sie oben definiert worden ist, bewirkt, und schließlich
d) eine Abspaltung der Schutzgruppen der Gruppe -CH(CH₂OH)₂ durchführt oder
e) eine Abspaltung der Schutzgruppen der Gruppe -CH(CH₂OH)₂ bewirkt und gegebenenfalls
f) eine Alkylierung der von den Schutzgruppen befreiten Verbindung mit einem Alkylierungsreagens R'₄Z durchführt.

7. Verfahren zur Herstellung eines Kontrastmittels, **dadurch gekennzeichnet**, daß man eine Verbindung der Formel I, wie sie in Anspruch 1 definiert worden ist, in eine verabreichbare Form bringt.
